# EUROPEAN PATENT APPLICATION

(11) **EP 3 992 273 A1**
(43) Date of publication of application: **04.05.2022**
(21) Application number: 20830864.3
(22) Date of filing: 25.06.2020
(51) Int. Cl.: C12M 1/00, C12N 5/071

(54) **CULTURE CONTAINER AND METHOD FOR PRODUCING REGENERATIVE HAIR FOLLICLE GERM USING CULTURE CONTAINER**

(30) Priority: 28.06.2019 JP 2019121816
(71) Applicant: RIKEN, Wako-shi Saitama 351-0198 (JP); Organ Technologies, Inc., Kobe-shi, Hyogo, 650-0047 (JP)
(72) Inventor: MORI Yutaka, Kyoto-shi, Kyoto 612-8501 (JP); KISHIMOTO Kyosuke, Kyoto-shi, Kyoto 612-8501 (JP); SAKAI Hisashi, Kyoto-shi, Kyoto 612-8501 (JP); UENO Ryo, Kyoto-shi, Kyoto 612-8501 (JP); TSUJI Takashi, Wako-shi, Saitama 351-0198 (JP); OKAMOTO Naokazu, Tokyo 105-0001 (JP); OGAWA Miho, Tokyo 105-0001 (JP)
(74) Representative: V.O.
(86) International application number: PCT/JP2020/025090
(87) International publication number: WO 2020/262554

(57) **Abstract**

A culture container (1) having a guide thread (31) and a plurality of cells (32) that is capable of culturing a regenerative hair follicle germ (30), wherein the culture container (1) is equipped with a first recess (10) and a second recess (20) and the second recess (20) has a second bottom (22) where the guide thread (31) can be positioned in the center of an opening (23) and a method for producing a regenerative hair follicle germ that includes a step for aggregating a cell population including epithelial cells (32a) within the second recess (20) of the culture container (1), a step for aggregating a cell population including mesenchymal cells (32b) within the second recess (20), a step for inserting the guide thread (31) into the second recess (20), and a step for culturing the cell population including epithelial cells (32a) and the mesenchymal cells (32b).

## Description

### CROSS-REFERENCE TO RELATED APPLICATION

The present application claims priority to Japanese Patent Application No. 2019-121816 filed on 28 June 2019, the content of which is hereby incorporated herein by reference in its entirety.

### TECHNICAL FIELD

The present disclosure relates to a culture container and to a method for producing a regenerative hair follicle germ using a culture container.

### BACKGROUND ART

As a treatment method for not only male pattern hair loss (AGA) but also congenital hair loss, cicatrix (scarring), dermatitis combustionis, female telogen phase hair loss, and other forms of alopecia, a treatment method is known in which epithelial stem cells and mesenchymal stem cells are separated from a small amount of hair follicular organs ingested from the normal scalp of the patient, a guide thread is inserted into an aggregated cell population comprising such stem cells to produce a regenerative hair follicle germ, and this regenerative hair follicle germ is grafted onto the site of the patient's alopecia (e.g., see Patent Reference No. 1).

With such a hair follicular regenerative treatment, because it is possible by culturing epithelial stem cells and mesenchymal stem cells separated from hair follicular organs to produce a large amount of regenerative hair follicle germs from a small amount of sample, this makes it possible to increase the total number of hairs (hair follicles) on the patient's scalp without increasing invasiveness.

### PRIOR ART REFERENCES

### PATENT REFERENCES

Patent Reference No. 1: International Patent Application Publication No. 2012/108069

### SUMMARY OF INVENTION

### PROBLEM TO BE SOLVED BY INVENTION

A culture container associated with an embodiment of the present disclosure permits culture of a regenerative hair follicle germ having a guide thread and a plurality of cells which are located at a tip portion of the guide thread, the culture container comprising a first concavity; and a second concavity which opens into a first base portion of the first concavity; wherein the second concavity has a second base portion which is capable of causing the guide thread to be positioned so as to appear to be located in a central portion of the opening when the opening is viewed from above.

Furthermore, a method for producing the regenerative hair follicle germ associated with an embodiment of the present disclosure is a method for producing the regenerative hair follicle germ comprising an operation in which a cell population comprising epithelial cells is made to agglomerate within the second concavity of any of the culture containers associated with the present disclosure; an operation in which a cell population comprising mesenchymal cells is made to agglomerate within the second concavity; an operation in which the guide thread in inserted within the second concavity; and an operation in which culture is carried out while the cell population comprising the agglomerated epithelial cells and the agglomerated mesenchymal cells are made to be in contact.

### BRIEF DESCRIPTION OF DRAWINGS

[FIG. 1] Vertical sectional view of culture container associated with an embodiment of the present disclosure.
[FIG. 2] Plan view of culture container shown in FIG. 1.
[FIG. 3] Enlarged sectional view of a portion at a second concavity in the culture container shown in FIG. 1.
[FIG. 4A] Drawing showing in schematic fashion how the culture container shown in FIG. 1 might be used to culture a regenerative hair follicle germ.
[FIG. 4B] Enlarged view of tip portion of guide thread at FIG. 4A.
[FIG. 5] Enlarged sectional view of a second concavity in accordance with a variation.
[FIG. 6A] Explanatory diagram showing a procedure for producing a regenerative hair follicle germ by means of a method for producing regenerative hair follicle germ associated with an embodiment of the present disclosure.
[FIG. 6B] Explanatory diagram showing a procedure for producing a regenerative hair follicle germ by means of a method for producing regenerative hair follicle germ associated with an embodiment of the present disclosure.
[FIG. 6C] Explanatory diagram showing a procedure for producing a regenerative hair follicle germ by means of a method for producing regenerative hair follicle germ associated with an embodiment of the present disclosure.
[FIG. 7] Drawing showing table containing hole diameters and depths of second concavities at culture containers used at working examples.
[FIG. 8] Drawing showing representative photographs of murine regenerative hair follicle germs, and regenerative hair follicle germ plating efficiency, produced using culture containers in accordance with working examples.
[FIG. 9] Drawing showing representative photographs of a murine regenerative hair follicle germ, and photographs of new hair growth following transplantation of the regenerative hair follicle germ, produced using Culture Container No. 3.
[FIG. 10] Drawing showing representative photographs of a murine regenerative hair follicle germ, and photographs of new hair growth following transplantation of the regenerative hair follicle germ, produced using Culture Container No. 4.

### EMBODIMENTS FOR CARRYING OUT INVENTION

Conventionally, populations of epithelial stem cells and mesenchymal stem cells separated from hair follicular organs are aggregated and made to be of high density and are partitioned, following which guide threads are inserted into said cell populations to produce regenerative hair follicle germs with guide threads. However, because regenerative hair follicle germs have conventionally been produced as a result of manual operations performed by humans, there has been much variation in the regenerative hair follicle germ finished product, and much time has been required for production thereof.

It is an object of the present disclosure to provide a culture container that will make it possible to easily and stably produce regenerative hair follicle germs and a method for producing regenerative hair follicle germs using such a culture container.

Embodiment in accordance with the present disclosure make it possible to provide a culture container that will make it possible to easily and stably produce regenerative hair follicle germs and a method for producing regenerative hair follicle germs using such a culture container.

Below, a culture container and a method for producing a regenerative hair follicle germ associated with an embodiment of the present disclosure are described in detail by way of example with reference to the drawings.

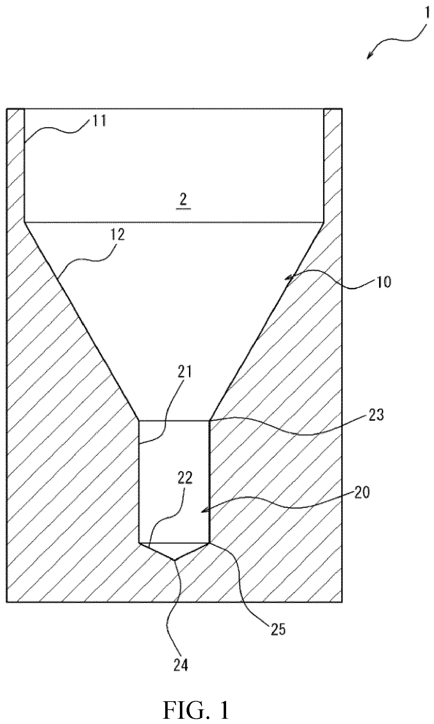

Note that what is referred to as the vertical direction in the present specification and claims should be understood to mean the vertical direction when the culture container is in the upright orientation shown in FIG. 1.

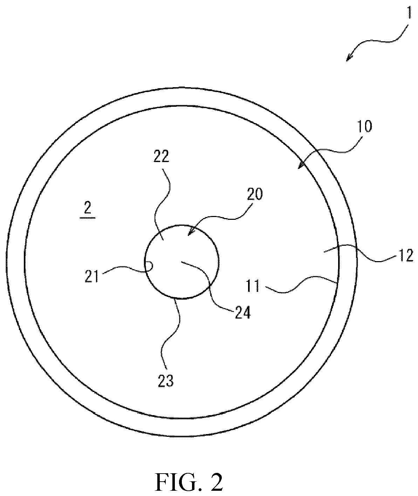

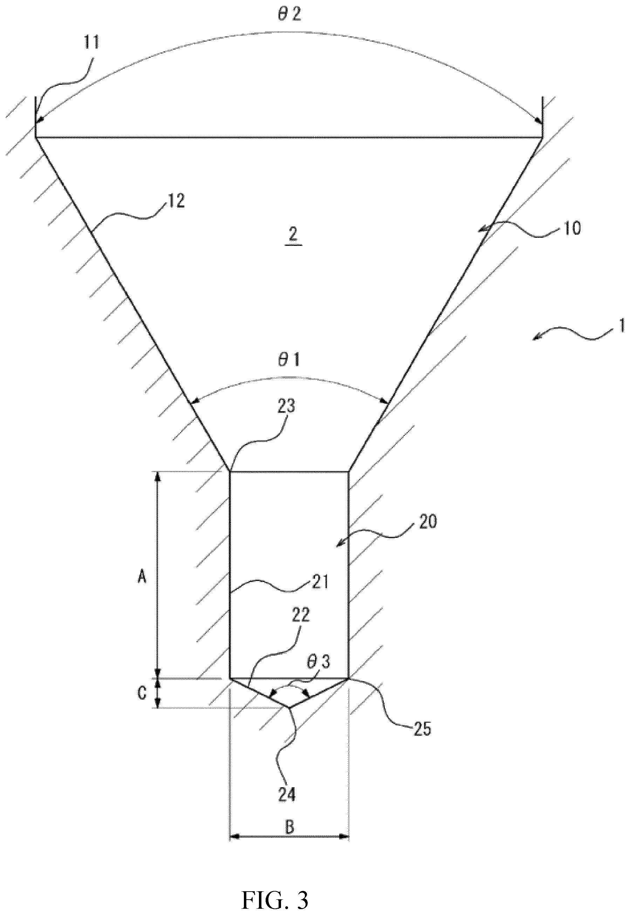

As shown in FIG. 1 through FIG. 3, culture container 1 associated with the present embodiment is provided with first concavity 10 and second concavity 20. First concavity 10 and second concavity 20 respectively constitute the containing space 2 of culture container 1.

Containing space 2 may, for example, be constituted such that it can be closed in liquid-tight fashion by a lid or other such closing member that is mounted in removably attachable fashion to the opening which is directed upward from culture container 1 (first concavity 10).

First concavity 10 is provided with first sidewall portion 11 and first base portion 12. First sidewall portion 11 has a cylindrical shape, the diameter (inside diameter) thereof being constant at all locations therealong in the vertical direction. First base portion 12 has a frustoconical shape, being inclined such that the diameter (inside diameter) thereof decreases as one proceeds toward the lower portion thereof, and is connected in integral fashion to the bottom end of first sidewall portion 11. Length in the vertical direction of first base portion 12 is greater than length in the vertical direction of first sidewall portion 11. Note that first sidewall portion 11 may also be inclined.

Length (depth) in the vertical direction of first concavity 10 might be 7 mm to 11 mm, length (depth) in the vertical direction of first sidewall portion 11 might be 4 mm to 6 mm, and length (depth) in the vertical direction of first base portion 12 might be 3 mm to 5 mm. In accordance with the present embodiment, length (depth) in the vertical direction of first concavity 10 is 9 mm, length (depth) in the vertical direction of first sidewall portion 11 is 5.1 mm, and length (depth) in the vertical direction of first base portion 12 is 3.9 mm. Furthermore, diameter of first sidewall portion 11 might be 4.0 mm to 5.5 mm, and is 4.9 mm in accordance with the present embodiment. Furthermore, the divergence angle θ1 of first base portion 12 might be 30° to 90°, and the divergence angle θ2 of first sidewall portion 11 might be 0° to 10°. In accordance with the present embodiment, the divergence angle θ1 of first base portion 12 is 60°, and the divergence angle θ2 of first sidewall portion 11 is 4°.

Whereas the constitution of the present embodiment is such that first concavity 10 is provided with first sidewall portion 11, where first base portion 12 is constituted so as to be inclined such that the diameter thereof decreases as one proceeds toward the lower portion thereof, the constitution may also be such that a first sidewall portion 11 is not provided thereat. Furthermore, whereas first base portion 12 of the present embodiment is of frustoconical shape such that the diameter thereof decreases linearly at a constant rate as one proceeds toward the lower portion thereof, there is no limitation with respect thereto, as the shape thereof may be any of various shapes, such as, for example, convex such that the diameter thereof gradually decreases as one proceeds toward the lower portion thereof, of curved shape so as to be concave, and so forth. In particular, where the constitution is such that first concavity 10 is provided with first sidewall portion 11, first base portion 12 may be planar in shape so as to be perpendicular to the vertical direction.

Second concavity 20 is provided with second sidewall portion 21 and second base portion 22. Second concavity 20 is provided below first concavity 10, and at second sidewall portion 21 this opens into the central portion of first base portion 12 of first concavity 10. Length (depth) in the vertical direction of second concavity 20 is less (less deep) than length (depth) in the vertical direction of first concavity 10.

Second sidewall portion 21 has a cylindrical shape, the diameter (inside diameter) thereof being constant at all locations therealong in the vertical direction, and the opening 23 thereof into first base portion 12 being circular. Furthermore, second sidewall portion 21 has a shape which is long in the vertical direction, length A thereof in the vertical direction being greater than diameter B of opening 23.

Second base portion 22 has a conical shape, being inclined such that the diameter thereof decreases as one proceeds toward the lower portion thereof, and is connected in integral fashion to the bottom end of second sidewall portion 21. When opening 23 of second concavity 20 is viewed from above (plan view), vertex 24 of second base portion 22 appears to be located in the central portion of second base portion 22. Connecting region 25 at which second sidewall portion 21 and second base portion 22 are connected is circular, diameter B of connecting region 25 being identical to the diameter of opening 23.

It is preferred that the length in the vertical direction of second base portion 22, i.e., depth C of second base portion 22, be less than diameter B of connecting region 25.

Furthermore, it is preferred that depth C of second base portion 22 be less than length A in the vertical direction of second sidewall portion 21.

Moreover, it is preferred that the surface roughness of second base portion 22 be greater than the surface roughness of second sidewall portion 21.

Length (depth) in the vertical direction of second concavity 20 might be 0.56 mm to 1.70 mm, length (depth) in the vertical direction of second sidewall portion 21 might be 0.5 mm to 1.5 mm, and length (depth) in the vertical direction of second base portion 22 might be 0.06 mm to 0.20 mm. In accordance with the present embodiment, length (depth) in the vertical direction of second concavity 20 is 1.0 mm, length (depth) in the vertical direction of second sidewall portion 21 is 0.9 mm, and length (depth) in the vertical direction of second base portion 22 is 0.09 mm. Furthermore, diameter B of second sidewall portion 21, i.e., connecting region 25, might be 0.3 mm to 1.0 mm, and is 0.42 mm in accordance with the present embodiment. Furthermore, the divergence angle θ3 of second base portion 22 might be 60° to 170°. In accordance with the present embodiment, the divergence angle θ3 of second base portion 22 is 120°.

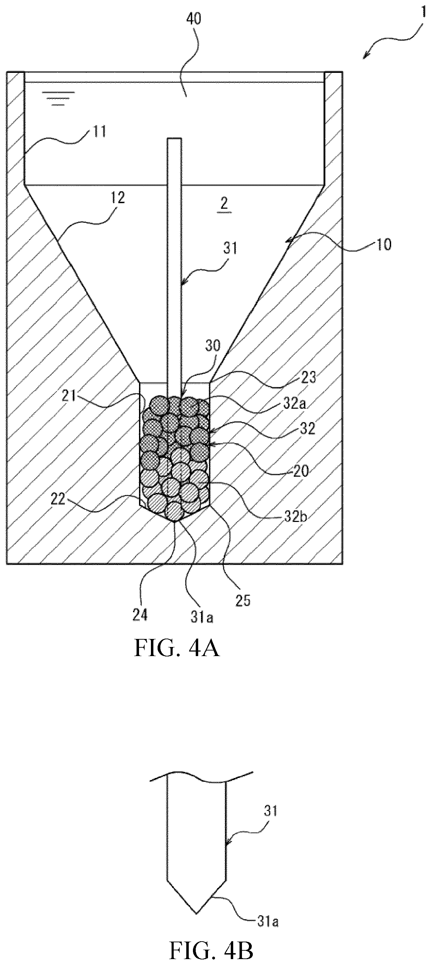

As shown in FIG. 4, culture container 1 might be such that culture of regenerative hair follicle germ 30 takes place at containing space 2. Regenerative hair follicle germ 30 has guide thread 31, and a plurality of cells 32 which are located at the tip portion of guide thread 31.

At the foregoing regenerative hair follicle germ 30, guide thread 31 may, for example, be such that nylon or other such polymer, stainless steel or other such metal, or carbon fiber, glass fiber, or other such chemical fiber or the like has been formed in the shape of a thread (fiber). In accordance with the present embodiment, guide thread 31 is made of nylon. As guide thread 31, a guide thread that is of smaller diameter than the diameter of second sidewall portion 21 of second concavity 20 (diameter B of connecting region 25) and that is of longer length than the length in the vertical direction of second concavity 20 may be employed.

The length of guide thread 31 might be 3 mm to 10 mm, and the diameter of guide thread 31 might be 0.02 mm to 1.20 mm. In accordance with the present embodiment, the length of guide thread 31 is 5 mm, and the diameter of guide thread 31 is 0.5 mm.

At the foregoing regenerative hair follicle germ 30, the plurality of cells 32 might, for example, be made up of a cell population comprising a plurality of epithelial cells 32a (indicated by crosshatching at FIG. 4) and a cell population comprising a plurality of mesenchymal cells 32b (indicated by hatching at FIG. 4). Where this is the case, it is preferred that the cell population comprising the plurality of mesenchymal cells 32b be arranged toward the tip 31a of guide thread 31 (toward second base portion 22) from the cell population comprising the plurality of epithelial cells 32a.

"Epithelial cells" means cells collected from epithelial tissue and cells obtained by culturing cells from collected cells, examples of which that may be cited including cells collected from the outermost layer of the outer root sheath at hair follicle-derived bulge regions (bulge region-derived epithelial cells), cells collected from adult or embryo skin epidermis, epithelial cells induced from pluripotent stem cells, hair follicles, epithelial precursor cells capable of differentiating into epithelial stem cells making up skin or stem cells thereof, and so forth.

"Mesenchymal cells" means cells collected from mesenchymal tissue and cells obtained by culturing cells from collected cells, examples of which that may be cited including dermal papilla-derived cells (e.g., dermal papilla cells), hair matrix cells, root sheath cells, inner root sheath cells, outer root sheath cells (exclusive of cells in outermost layer), adult or embryo skin mesenchymal cells, hair follicular mesenchymal cells induced from pluripotent stem cells, bone marrow cells not including blood cells, bone marrow-derived mesenchymal cells, mandibular bone marrow cells, mesenchymal cells derived from cephalic neural crest cells or mesenchymal precursor cells capable of differentiating into such cells, stem cells, and so forth.

"Pluripotent stem cells" means cells having ability to differentiate into any of various types of tissue possessed by an organism, examples of which that may be cited including ES cells (embryonic stem cells), ntES cells (nuclear transfer embryonic stem cells), EG cells (embryonic germ cells), GS cells (germline stem cells), and iPS cells (induced pluripotent stem cells).

Epithelial cells and mesenchymal cells used for manufacturing regenerative hair follicle germs may be collected from various types of animals and the like including mammals such as humans, monkeys, pigs, cows, horses, mice, rats, guinea pigs, rabbits, dogs, cats, and so forth.

Epithelial cells and mesenchymal cells from hair follicles, skin, and/or other such tissue may be surgically collected therefrom or may be collected therefrom as a result of reaction with dispase, collagenase, trypsin, and/or other such enzyme(s) to cause separation into epithelial tissue and mesenchymal tissue, and reaction of the separated epithelial tissue and mesenchymal tissue with dispase, collagenase, trypsin, and/or other such enzyme(s). Enzyme reaction time should be varied as appropriate depending on the size of the tissue, properties (e.g., proteins contributing to intercellular junctions, strength of intercellular junctions , etc.) of the cells which make up the tissue, and so forth. Furthermore, two or more enzymes may be used in combination during reaction for separation into epithelial tissue and mesenchymal tissue or for collection of epithelial cells and mesenchymal cells from epithelial tissue and mesenchymal tissue.

"Cell population" means a cell suspension in which cells are in a state in which they are distributed in a liquid, such as that which may be prepared by causing collected epithelial cells and/or mesenchymal cells and/or cells cultured from such cells to be added to a culture medium such as will permit culture of said cells, and carrying out mixing so as to cause these to be distributed therein.

To obtain a large amount of epithelial cells and/or mesenchymal cells, culture of said cells may be carried out. Where culture of said cells is carried out, as culture medium used to carry out culture thereof, it is sufficient that this be a basal medium such as may be employed for culture of animal cells, examples of which that may be cited including Dulbecco's Modified Eagle's Medium (DMEM), Advanced DMEM/F12 Medium, and so forth. Furthermore, for efficient growth of epithelial cells and/or mesenchymal cells, blood serum, FGF (fibroblast growth factor), EGF (epidermal growth factor), and/or other such cell growth factor(s) may be added. Furthermore, as additives for maintenance of cell function, Y27632, Noggin, SAG, and/or the like may be added, and Penicillin, Streptomycin, and/or other such antibiotic(s) may be added as necessary. Basal medium, blood serum, cell growth factor(s), and additive(s) should be varied as appropriate depending on the properties of the epithelial cells and/or mesenchymal cells. It is sufficient that cell culture conditions be such as to create an environment that will permit cell growth to occur, it being possible, for example, to employ culture which is carried out in an incubator having a 5% concentration of CO₂ and a temperature of 37° C.

As the cell population comprising a plurality of epithelial cells 32a, it is preferred that a cell population comprising bulge region epithelium-derived cells be employed. Furthermore, as the cell population comprising a plurality of mesenchymal cells 32b, it is preferred that a cell population comprising dermal papilla-derived cells be employed.

Because regenerative hair follicle germ 30 having the foregoing constitution is constituted such that a plurality of cells 32 are located at the tip portion of guide thread 31, because it will be possible, when grafting the plurality of cells 32 to the interior of the scalp by way of an incised portion provided at the scalp, to use tweezers to grasp guide thread 31, handling during transplantation is facilitated. Furthermore, by causing the plurality of cells 32 to be grafted to the scalp in such fashion that guide thread 31 is made to protrude outside the scalp, it will be possible, where the plurality of cells 32 is made up of a cell population comprising a plurality of epithelial cells 32a and a cell population comprising a plurality of mesenchymal cells 32b, to easily graft the plurality of cells 32 so as to cause the cell population comprising the epithelial cells 32a to be located toward the surface of the scalp and so as to cause the cell population comprising the mesenchymal cells 32b to be located toward the interior of the scalp. Moreover, by improving continuity between epithelial cells 32a making up regenerative hair follicle germ 30 and epithelial cells in scalp skin tissue following transplantation, guide thread 31 promotes new hair growth effect.

When using culture container 1 in accordance with the present embodiment to culture regenerative hair follicle germ 30, containing space 2 is filled with culture solution 40, the plurality of cells 32 are contained within second concavity 20, guide thread 31 is made to come in contact with second base portion 22 at downwardly directed tip 31a, and the portion toward the other end thereof which is directed upward is made to protrude upward from cells 32.

Here, at culture container 1 in accordance with the present embodiment, second base portion 22 of second concavity 20 is constituted so as to be capable of causing guide thread 31 of regenerative hair follicle germ 30 which is contained within containing space 2 to be positioned so as to appear to be located in the central portion of opening 23 when opening 23 is viewed from above (plan view). That is, second base portion 22 is capable of facilitating positioning of guide thread 31 of regenerative hair follicle germ 30 so that it is arranged along the axis of second concavity 20 when regenerative hair follicle germ 30 is contained within containing space 2.

In accordance with the present embodiment, second base portion 22 has a conical shape, being inclined such that the diameter thereof decreases as one proceeds toward the lower portion thereof, the shape thereof being such that vertex 24 which appears to be located in the central portion thereof as viewed from above is downwardly directed and is the most deeply recessed region thereof. Accordingly, when regenerative hair follicle germ 30 is contained within containing space 2 of culture container 1, this will make it possible to reduce movement in the radial direction with respect to vertex 24 of tip 31a of guide thread 31, and will make it possible to facilitate positioning of guide thread 31 so that it appears to be located in the central portion of opening 23 when opening 23 is viewed from above.

Thus, at culture container 1 in accordance with the present embodiment, because second concavity 20 has second base portion 22 which is capable of causing guide thread 31 to be positioned so as to appear to be located in the central portion of opening 23 when opening 23 is viewed from above, it will be possible, when causing culture of regenerative hair follicle germ 30 having guide thread 31 and the plurality of cells 32 located at the tip portion of guide thread 31 to be carried out at containing space 2, to cause said guide thread 31 to be positioned in the central portion of second concavity 20. Because this makes it possible to cause guide thread 31 to be positioned so that it is arranged along the axis of second concavity 20, this will make it possible to easily and stably produce regenerative hair follicle germ 30.

Furthermore, at culture container 1 in accordance with the present embodiment, because second base portion 22 is constituted so as to be inclined such that the diameter thereof decreases as one proceeds toward the lower portion thereof, this makes it possible, when inserting guide thread 31 into the interior of second concavity 20, for tip 31a of guide thread 31 to be guided along the incline of second base portion 22 and into the central portion of second base portion 22, permitting guide thread 31 to be even more easily positioned within the central portion of opening 23.

In particular, where, as is the case in the present embodiment, second base portion 22 is made to be of conical shape, it will be possible, when inserting guide thread 31 into the interior of second concavity 20, for tip 31a of guide thread 31 to be guided by vertex 24, permitting guide thread 31 to be positioned within the central portion of opening 23.

Whereas the present embodiment is such that second concavity 20 is provided with second sidewall portion 21, where second base portion 22 is constituted so as to be inclined such that the diameter thereof decreases as one proceeds toward the lower portion thereof, the constitution may also be such that a second sidewall portion 21 is not provided thereat. Furthermore, whereas second base portion 22 of the present embodiment is of conical shape such that the diameter thereof decreases as one proceeds toward the lower portion thereof, there is no limitation with respect thereto, as the shape thereof may be any of various shapes, such as, for example, convex such that the diameter thereof gradually decreases as one proceeds toward the lower portion thereof, of curved shape so as to be concave, and so forth, so long it will permit guide thread 31 to be positioned so as to appear to be located in the central portion of opening 23 when opening 23 is viewed from above.

Because first base portion 12 of the present embodiment is constituted so as to be inclined such that the diameter thereof decreases as one proceeds toward the lower portion thereof, it will be possible, when regenerative hair follicle germ 30 is to be contained within containing space 2, for tip 31a of guide thread 31 and the plurality of cells 32 to be guided along first base portion 12 and into second concavity 20, making it possible to cause tip 31a of guide thread 31 and the plurality of cells 32 to be easily made to be contained within second concavity 20.

Culture container 1 having the foregoing constitution might, for example, be made of polystyrene. In such case, culture container 1 may be formed by cutting from polystyrene stock, or it may be formed by using a die to carry out injection molding. Where a die is used to carry out injection molding of polystyrene to fabricate culture container 1, this will facilitate mass production of culture container 1.

Note that culture container 1 is not limited to being made of polystyrene but may, for example, be formed from Teflon (registered trademark), polypropylene, polyvinyl chloride, polyethylene, cyclic polyolefin, polyethylene terephthalate, polycarbonate, polydimethylsiloxane, polymethyl methacrylate, polyarylate, polysulfone, polyether sulfone, polyether ether ketone, polyether imide, polymethylpentene, silicone resin, acrylic, PFA, and/or other such resin material(s), and/or may be formed from glass and/or other such material(s).

At culture container 1 having the foregoing constitution, because second concavity 20 is of such shape that length A in the vertical direction of second sidewall portion 21 is greater than diameter B of opening 23, it will be possible to cause the plurality of cells 32 being cultured at second concavity 20 to be cultured in such fashion as to have a shape which is suitable for new hair growth. Note that it is also possible for second concavity 20 to be of such shape that length A in the vertical direction of second sidewall portion 21 is of the same length as diameter B of opening 23, and it is also possible for it to be of such shape that length A in the vertical direction of second sidewall portion 21 is less than diameter B of opening 23.

Furthermore, at culture container 1 having the foregoing constitution, because second concavity 20 is of such shape that length A in the vertical direction of second sidewall portion 21 is greater than depth C of second base portion 22, it will be possible to cause the plurality of cells 32 being cultured at second concavity 20 to be cultured in such fashion as to have a shape which is suitable for new hair growth in that the base portion which is toward tip 31a of guide thread 31 is rounded. Note that it is also possible for second concavity 20 to be of such shape that length A in the vertical direction of second sidewall portion 21 is of the same length as depth C of second base portion 22, and it is also possible for it to be of such shape that length A in the vertical direction of second sidewall portion 21 is less than depth C of second base portion 22.

Moreover, at culture container 1 having the foregoing constitution, because second concavity 20 is of such shape that depth C of second base portion 22 is less than diameter B of connecting region 25, it will be possible to cause the plurality of cells 32 being cultured at second concavity 20 to be stably cultured in such fashion as to have a shape which is suitable for new hair growth and at which the base portion that is toward tip 31a of guide thread 31 is rounded. Note that it is also possible for second concavity 20 to be of such shape that depth C of second base portion 22 is of the same length as diameter B of connecting region 25, and it is also possible for it to be of such shape that depth C of second base portion 22 is greater than diameter B of connecting region 25.

Moreover, at culture container 1 having the foregoing constitution, because the surface roughness of second base portion 22 is greater than the surface roughness of second sidewall portion 21, it will be possible to cause the plurality of cells 32 being cultured at second concavity 20 to be cultured in stable fashion. Note that it is also possible for second concavity 20 to be constituted such that the surface roughness of second base portion 22 is identical to the surface roughness of second sidewall portion 21, and it is also possible for it to be constituted such that the surface roughness of second base portion 22 is less than the surface roughness of second sidewall portion 21.

At culture container 1 having the foregoing constitution, because the shapes of first concavity 10 and second concavity 20 are suitable for production of regenerative hair follicle germ 30, it will be possible to produce a regenerative hair follicle germ 30 of such shape that the plurality of cells 32 are suitable for new hair growth.

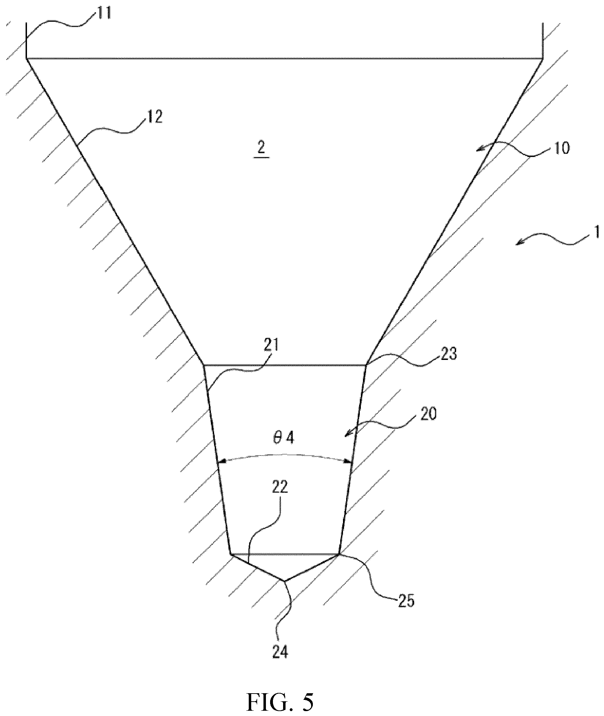

FIG. 5 is an enlarged sectional view of second concavity 20 in accordance with a variation.

As shown in FIG. 5, second concavity 20 may also be constituted such that second sidewall portion 21 is inclined such that the diameter thereof decreases as one proceeds toward the lower portion thereof. In such case, the constitution of culture container 1 is made to be such that first base portion 12, second sidewall portion 21, and second base portion 22 are respectively inclined with respect to the vertical direction, and is made to be such that the angles of first base portion 12 and second base portion 22 with respect to the vertical direction are greater than the angle of second sidewall portion 21 with respect to the vertical direction. Moreover, the divergence angle θ4 of second sidewall portion 21 might be 0° to 30°. In accordance with the present embodiment, the divergence angle θ4 of second sidewall portion 21 is 2°.

Such a constitution will make it possible for tip 31a of guide thread 31 and the plurality of cells 32 to be guided along first base portion 12 which is inclined such that the diameter thereof decreases as one proceeds toward the lower portion thereof until second concavity 20 is reached, and to be guided along second sidewall portion 21 which is inclined such that the diameter thereof decreases as one proceeds toward the lower portion thereof until second base portion 22 is reached, and for tip 31a of guide thread 31 to easily be positioned in the central portion of second base portion 22, and for the plurality of cells 32 to be easily made to be contained within second concavity 20.

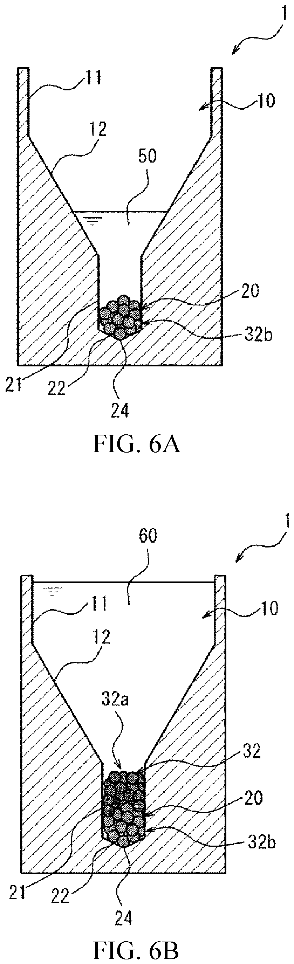

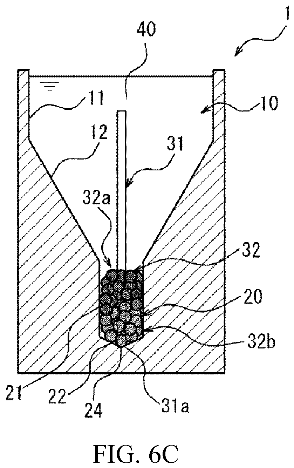

Next, a method for producing regenerative hair follicle germ 30 using culture container 1 in accordance with the present embodiment will be described with reference to FIG. 6.

At a method for producing a regenerative hair follicle germ in accordance with the present embodiment, an operation is first carried out in which a cell population comprising mesenchymal cells 32b is made to agglomerate within second concavity 20 of culture container 1. At said operation, containing space 2 of culture container 1 is filled with an appropriate amount (10 µL to 100 µL) of cell suspension 50 (cell density = 1×10⁵ cells/mL to 2×10⁶ cells/mL) comprising a plurality of mesenchymal cells, and centrifugation (1 min to 10 min at 500×g to 700×g) is carried out to cause the plurality of mesenchymal cells 32b to agglomerate (number of cells = 1×10³ cells to 2×10⁵ cells) toward second base portion 22 of second concavity 20. At said operation, note that it is sufficient that the amount of the cell suspension which is added not be an amount such as will cause the container to overflow, and note with respect to centrifugation conditions as well that it is sufficient that conditions be such as not to impair cell function. As shown in FIG. 6A, this will cause a cell population comprising a plurality of mesenchymal cells 32b to agglomerate so as to be adjacent to second base portion 22 within second concavity 20.

Next, an operation is carried out in which a cell population comprising epithelial cells 32a is made to agglomerate within second concavity 20 of culture container 1. At said operation, following removal of the liquid supernatant at the cell suspension 50 comprising the plurality of mesenchymal cells, containing space 2 of culture container 1 is filled with an appropriate amount (10 µL to 100 µL) of cell suspension 60 (cell density = 1×10⁵ cells/mL to 4×10⁶ cells/mL) comprising a plurality of epithelial cells, and centrifugation (1 min to 10 min at 500×g to 700×g) is carried out to cause the plurality of epithelial cells 32a to agglomerate (number of cells = 1×10³ cells to 4×10⁵ cells) in layered fashion above mesenchymal cells 32b at second concavity 20. At said operation, it is sufficient that the amount of the cell suspension which is added not be an amount such as will cause the container to overflow, and with respect to centrifugation conditions as well that it is sufficient that conditions be such as not to impair cell function. As shown in FIG. 6B, this will cause a cell population comprising a plurality of epithelial cells 32a to agglomerate in layered fashion above mesenchymal cells 32b within second concavity 20. Note that the liquid supernatant at the cell suspension 50 need not be removed.

Next, as shown in FIG. 6C, guide thread 31 is inserted in second concavity 20. Following insertion within second concavity 20, guide thread 31 is downwardly directed from a location thereabove above as it is made to penetrate the cell populations comprising mesenchymal cells 32b and epithelial cells 32a, and tip 31a which is directed downward therefrom is made to abut second base portion 22. Because as described above second base portion 22 of second concavity 20 is constituted so as to be capable of causing guide thread 31 to be positioned so as to appear to be located in the central portion of opening 23 when opening 23 is viewed from above, second base portion 22 causes guide thread 31, tip 31a of which is made to come in contact with second base portion 22, to be positioned such that tip 31a is aligned with vertex 24, and to be arranged such that it appears to be located in the central portion of opening 23 when opening 23 is viewed from above. This causes guide thread 31 to be arranged so as to penetrate the central portions of the cell populations comprising mesenchymal cells 32b and epithelial cells 32a.

Next, the cell population comprising epithelial cells 32a agglomerated at the foregoing operations and mesenchymal cells 32b agglomerated thereat may be cultured while they are made to be in mutual contact within second concavity 20. It is sufficient that culture conditions at said culture be such as will permit interaction therebetween while maintaining functionality of the epithelial cells and the mesenchymal cells, it being possible, for example, for culture to be carried out for 12 hours to 36 hours within an incubator having a 5% concentration of CO₂ and a temperature of approximately 37° C.

In accordance with such a method for producing a regenerative hair follicle germ, a plurality of cells 32 comprising agglomerated mesenchymal cells 32b and agglomerated epithelial cells 32a are made to be uniformly positioned about the tip portion of guide thread 31, making it possible to easily and stably produce a regenerative hair follicle germ 30 which is of such shape as to be suitable for new hair growth.

By, for example, using a culture container 1 at which length A in the vertical direction of second sidewall portion 21 is 900 µm ± 10 µm, and at which diameter B of connecting region 25 and opening 23 of second concavity 20 is 450 µm ± 10 µm, this will make it possible to facilitate easy and stable production of regenerative hair follicle germ 30.

Whereas in accordance with the present embodiment the operation in which the cell population comprising mesenchymal cells 32b is made to agglomerate is carried out before the operation in which the cell population comprising epithelial cells 32a is made to agglomerate within second concavity 20 of culture container 1 is carried out, it is also possible to carry out the operation in which the cell population comprising epithelial cells 32a is made to agglomerate within second concavity 20 of culture container 1 and thereafter carry out the operation in which the cell population comprising mesenchymal cells 32b is made to agglomerate.

Furthermore, whereas in accordance with the present embodiment the operation in which guide thread 31 is inserted is carried out after the operations in which the cell population comprising epithelial cells 32a and the cell population comprising mesenchymal cells 32b are made to agglomerate, it is also possible to first insert guide thread 31 in second concavity 20 and cause said guide thread 31 to be positioned in the central portion of second concavity 20 by second base portion 22 and to thereafter carry out the operations in which the cell population comprising epithelial cells 32a and the cell population comprising mesenchymal cells 32b are made to agglomerate. In this case as well, it will also be possible to easily and precisely produce a regenerative hair follicle germ 30 in which cell populations comprising mesenchymal cells 32b and epithelial cells 32a are uniformly positioned about the tip portion of guide thread 31.

Whereas in accordance with the present embodiment a cell population comprising bulge region epithelium-derived cells was employed as the cell population comprising epithelial cells 32a, there is no limitation with respect thereto, it also being possible to employ other cell population(s) as the cell population comprising epithelial cells 32a. Furthermore, whereas in accordance with the present embodiment a cell population comprising dermal papilla-derived cells was employed as the cell population comprising mesenchymal cells 32b, there is no limitation with respect thereto, it also being possible to employ other cell population(s) as the cell population comprising mesenchymal cells 32b.

### WORKING EXAMPLES

Below, the present invention is described in further detail with reference to working examples. Note, however, that as the present invention is capable of being embodied in a wide variety of modes in which any manner of variations are possible with respect to the number of cells that are seeded, the timing with which medium is replaced, culture time, reagents employed, reagent concentrations, enzyme reaction time, and so forth, it should not be construed as being limited by the working examples that are described herein.

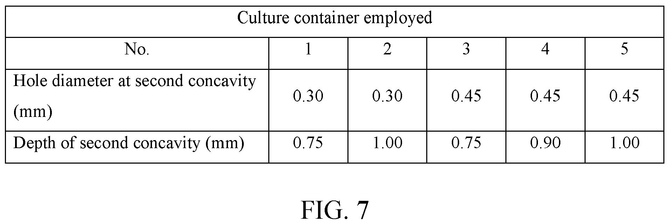

As shown in FIG. 7, culture containers were prepared in accordance with working examples which respectively had constitutions as described above and in which Container No. 1 was such that hole diameter (diameter of the opening) at the second concavity was 0.30 mm and depth of the second concavity was 0.75 mm, Container No. 2 was such that hole diameter at the second concavity was 0.30 mm and depth of the second concavity was 1.00 mm, Container No. 3 was such that hole diameter at the second concavity was 0.45 mm and depth of the second concavity was 0.75 mm, Container No. 4 was such that hole diameter at the second concavity was 0.45 mm and depth of the second concavity was 0.90 mm, and Container No. 5 was such that hole diameter at the second concavity was 0.45 mm and depth of the second concavity was 1.00 mm. Furthermore, for each of the respective containers, a (cut) container formed by cutting from polystyrene or Teflon (registered trademark) was prepared.

### Working Example 1: Use of Mice for Production of Regenerative Hair Follicle Germ and Evaluation of Organ Inductivity

### (1) Preparation of Reagents

### (1-1) Preparation of Culture Medium for Dissection

FBS (manufactured by Biowest; final concentration = 10%), HEPES (manufactured by Thermo Fisher Scientific; final concentration = 10 mM), and Penicillin-Streptomycin (manufactured by Thermo Fisher Scientific; final concentration = 1%) were mixed in DMEM (manufactured by Thermo Fisher Scientific).

### (1-2) Preparation of Culture Medium for Culture of Murine Dermal Papilla Cells

FBS (final concentration = 10%), Penicillin-Streptomycin (final concentration = 1%), and bFGF (manufactured by Fujifilm Wako Pure Chemical Corporation; final concentration = 10 ng/mL) were added to DMEM, and this was thereafter mixed.

### (1-3) Preparation of 0.05% Trypsin-EDTA

This was prepared by diluting 0.5% Trypsin - 2 g/L EDTA·4Na (manufactured by Sigma-Aldrich) to 10× in PBS (-) (manufactured by Nacalai Tesque, Inc.) and mixing.

### (1-4) Preparation of Culture Medium for Recovery of Murine Dermal Papilla Cells

FBS (final concentration = 10%) and Penicillin-Streptomycin (final concentration = 1%) were added to DMEM, and this was thereafter mixed.

### (1-5) Preparation of Separation Enzyme Solution

Collagenase I (manufactured by Worthington; final concentration = 50 U/mL) was added to Dispase (manufactured by Becton, Dickinson and Company; concentration = 50 caseinolytic U/mL), and this was mixed.

### (1-6) Preparation of Disaggregation Enzymatic Reaction Solution

2.5% Trypsin (manufactured by Thermo Fisher Scientific) was diluted to 50x in PBS (-), and this was mixed (final concentration = 0.05%).

### (1-7) Preparation of NFFSE Culture Medium

HEPES (manufactured by Thermo Fisher Scientific; final concentration = 10 mM), Glutamax (manufactured by Thermo Fisher Scientific; final concentration = 1×), B27 supplement (manufactured by Thermo Fisher Scientific; final concentration = 1×), N2 supplement (manufactured by Thermo Fisher Scientific; final concentration = 1×), Rock inhibitor (Y27632; manufactured by Fujifilm Wako Pure Chemical Corporation; final concentration = 10 µM), EGF (manufactured by Peprotech; final concentration = 50 ng/mL), FGF-7 (manufactured by R&D Systems; final concentration = 50 ng/mL), FGF-10 (manufactured by R&D Systems; final concentration = 50 ng/mL), SHH agonist (SAG; manufactured by Cayman Chemical Company; final concentration = 50 ng/mL), and BMP inhibitor (Noggin; manufactured by Peprotech; 50 ng/mL), as well as Penicillin-Streptomycin (final concentration = 1%) were added to Advanced DMEM/F-12 (manufactured by ThermoFisher), and this was mixed.

### (1-8) Preparation of HEPES

HEPES (manufactured by Dojindo (Dojindo Laboratories)) was dissolved in MilliQ water, and NaOH (manufactured by Fujifilm Wako Pure Chemical Corporation) was thereafter used to adjust pH so as to be 7.4 to prepare 1 M HEPES.

### (1-9) Preparation of NaHCO₃

NaHCO₃ (manufactured by Fujifilm Wako Pure Chemical Corporation) was dissolved in MilliQ water to prepare 1 M NaHC03.

### (1-10) Preparation of DMEM

Powder (manufactured by Thermo Fisher Scientific) for 1 L of DMEM was dissolved in 100 mL of MilliQ water to prepare 10× DMEM.

### (2) Culture of Murine Dermal Papillae cell

### (2-1) Collection and Seeding of Murine Dermal Papillae Tissues

C57BL/6 mice (SLC, Inc.) and C57BL/6-TgN (act-EGFP) mice (SLC, Inc.) of age 7-8 weeks were euthanized by cervical dislocation, following which scissors were used to remove the whiskers therefrom. Scissors were used to collect whisker tissue in a manner such as would not cause damage to the hair bulb. Whisker tissue was disinfected and washed twice in each of Isodine (manufactured by Meiji Seika Pharma), PBS (-), and culture medium for dissection (see above). Following removal of subcutaneous tissue from whisker tissue while in the culture medium for dissection, whisker hair follicles were pulled out from whisker tissue to recover whisker hair follicles. Following selection of whisker hair follicles in anagen stages I through IV, a scalpel was used to cut the top portion of the hair bulb, and a 25G injection needle was used to extract the dermal papilla from the hair bulb. Note that the extracted dermal papillae tissues were recovered in culture medium for dissection. The recovered dermal papillae tissues were seeded in a 35 mm culture dish (manufactured by Becton, Dickinson and Company) or a 60 mm culture dish (manufactured by Becton, Dickinson and Company), and were cultured for 6-8 days in culture medium for culture of murine dermal papilla cells (see above).

### (2-2) Recovery of Murine Dermal Papilla Cells

Following culture for 6-8 days, the supernatant was discarded, and washing was carried out twice with PBS (-). Following washing, 0.05% Trypsin-EDTA (see above) was added thereto, and after this had pervaded the entirety, this was allowed to react for 5 minutes at 37° C. Reaction was stopped by adding culture medium for recovery of murine dermal papilla cells (see above), and cells were recovered to prepare recovered cell solution. The recovered cell solution was centrifuged for 5 minutes at 4° C and 250×g, the supernatant was discarded, and this was thereafter suspended in culture medium for recovery of murine dermal papilla cells to prepare murine dermal papilla cell suspension. Following preparation thereof, cell concentration was measured. Culture medium for recovery of murine dermal papilla cells was used to prepare a murine dermal papilla cell suspension in which there were 1×10⁴ cells per 10 µL to 50 µL.

### (3) Culture of Murine Epithelial Cells

### (3-1) Collection and Seeding of Murine Epithelial Cells

A scalpel was used to cut the ringwulst of the whisker hair follicle, and the collagen sheath was removed to separate bulge region tissue therefrom. The bulge region tissue was recovered into a 35 mm Petri dish (manufactured by Becton, Dickinson and Company) to which culture medium for dissection had been added. Following removal of the culture medium for dissection and addition of separation enzyme solution (see above) (1 mL being added to approximately 150 bulge regions worth of bulge region tissue), this was allowed to react for 5 minutes in a CO₂ incubator (manufactured by Panasonic Healthcare Co., Ltd.) set to have a 5% concentration of CO₂ and set to be at a temperature of 37° C. Following reaction, this was washed twice with culture medium for dissection, 2 mL of culture medium for dissection which contained 35 U/mL DNase Type I (manufactured by Sigma-Aldrich) was added thereto, and a 25G injection needle was thereafter used to surgically remove the mesenchymal tissue which surrounded the bulge region tissue to collect bulge region epithelial tissue. After washing the bulge region epithelial tissue twice with PBS (-), 2 mL of disaggregation enzymatic reaction solution (see above) was added to approximately 150 bulge regions worth of bulge region epithelial tissue, and this was allowed to react for 1 hour in a CO₂ incubator set to have a 5% concentration of CO₂ and set to be at a temperature of 37° C. Following reaction, 1 mL of culture medium for dissection which contained 70 U/mL DNase Type I was added to stop the enzymatic reaction, this was mixed by pipetting, this was made to pass through a 35-µm cell strainer (manufactured by Corning), and this was centrifuged for 3 minutes at 4° C and 310×g. Following centrifugation, culture medium for dissection was added and this was mixed to prepare murine epithelial cells. Following measurement of cell concentration of the murine epithelial cells, cell suspension aliquots were transferred to tubes. The tubes were centrifuged for 3 minutes at 4° C and 310xg, the supernatant was discarded, and a 1% Atelocollagen solution (Koken) which contained HEPES (manufactured by Dojindo (Dojindo Laboratories); final concentration = 10 mM), NaHCO₃ (see above; final concentration = 10 mM), and DMEM (see above; final concentration = 1×) was added thereto and this was mixed to prepare a 5×10⁴ cells/mL to 1×10⁵ cells/mL cell suspension. Following centrifugation to remove bubbles, the wells of a 6-well plate (manufactured by Becton, Dickinson and Company) were respectively seeded with 90 µL/well of the cell suspension. After allowing the gel to solidify for on the order of 30 minutes within a CO₂ incubator set to have a 5% concentration of CO₂ and set to be at a temperature of 37° C, 3 mL/well of NFFSE culture medium was added thereto, and three-dimensional culture was carried out for 5-6 days within a CO₂ incubator set to have a 5% concentration of CO₂ and set to be at a temperature of 37° C.

### (3-2) Recovery of Murine Epithelial Cells

Following culture for 5-6 days, a cell scraper was used to cause the gel at which cells had formed colonies to be scraped out of the culture dish. The gel that was scraped therefrom was recovered in a 1.5-mL tube (such that there were a maximum of 3 gel clumps per tube), and 1 mL of the culture supernatant was added thereto. Collagenase I was added to obtain a final concentration of 100 U/mL, and this was allowed to react for 60-90 minutes at 37° C to dissolve the gel. Following centrifugation for 3 minutes at 4° C and 590×g, the supernatant was discarded, and washing was carried out once with 1 mL of PBS (-). Following washing, 500 µL of 0.125% Trypsin (manufactured by Thermo Fisher Scientific) which had been diluted using PBS (-) was added thereto, and this was allowed to react for 20 minutes at 37° C. 1 mL of culture medium for dissection which contained 35 U/mL DNase Type I was added thereto and this was mixed to prepare a cell suspension. The cell suspension was made to pass through a 35-µm cell strainer, and this was centrifuged for 3 minutes at 4° C and 590×g. Following centrifugation, the supernatant was discarded, and this was suspended in Advanced DMEM/F-12 culture medium which contained 1% Penicillin-Streptomycin to prepare a murine epithelial cell suspension. Following preparation thereof, cell concentration was measured. NFFSE culture medium was used to prepare a murine epithelial cell suspension in which there were 1×10⁴ cells to 2×10⁴ cells per 20 µL to 50 µL.

### (4) Washing of Culture Containers (Containers for Reconstruction)

The cut culture containers No. 1 through No. 5 were respectively immersed in 70% ethanol in which 99.5% ethanol (manufactured by Fujifilm Wako Pure Chemical Corporation) had been diluted with MilliQ water, these were ultrasonically treated for 30 minutes at room temperature, and a stereoscopic microscope (manufactured by Carl Zeiss) was used to determine whether residue remained from cutting. The culture containers were immersed in MilliQ water to which neutral detergent (manufactured by Kao Corporation) had been added (7 drops of neutral detergent being added per 50 mL of MilliQ water), and these were washed for 3 hours at room temperature in a shaker (60 rpm). Following washing, the culture containers were washed in running water for 1 hour, following which (determination as to whether residue remained from cutting being carried out by stereoscopic microscopy at 30 minutes following the start of washing in running water) these were immersed in MilliQ water, and these were washed for 1 hour at room temperature in a shaker (60 rpm). Note that deaeration was carried out by pipetting. The MilliQ water was replaced, and washing was carried out overnight at room temperature in a shaker (60 rpm). The MilliQ water was again replaced, and washing was carried out for 1 hour at room temperature in a shaker (60 rpm). These were disinfected twice with 70% ethanol, and these were further disinfected twice with 99.5% ethanol (deaeration being carried out by pipetting). The culture containers were air-dried in Petri dishes within a biological safety cabinet (manufactured by Sanyo).

### (6) Manufacture of Regenerative Hair Follicle Germ

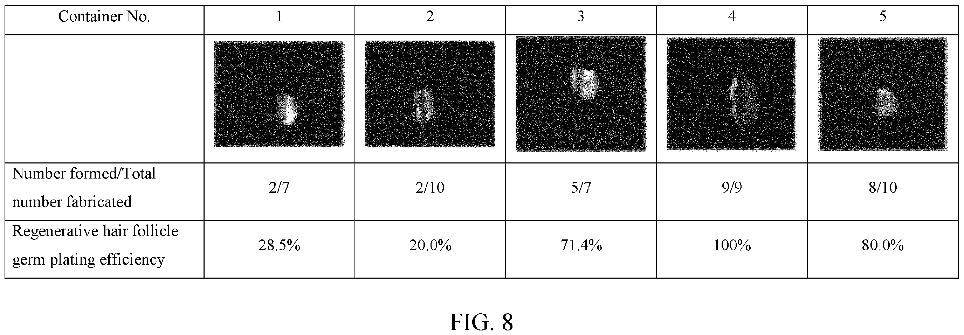

The cut culture containers No. 1 through No. 5 were used to manufacture regenerative hair follicle germs. The respective washed culture containers were inserted in a 96-well plate (manufactured by Becton, Dickinson and Company). At each of the respective culture containers, culture medium for culture of murine dermal papilla cells was added until the top portion of the second concavity was reached, following which 10 µL to 50 µL of murine dermal papilla cell suspension was added to the culture container (number of cells = 1×10⁴ cells per container), and this was centrifuged at room temperature for 3-5 minutes at 590×g. Following centrifugation, supernatant was discarded until the top portion of the second concavity was reached, 20 µL to 50 µL of murine epithelial cell suspension was added to the culture container (number of cells = 1×10⁴ cells to 2×10⁴ cells per container), and this was centrifuged at room temperature for 3-5 minutes at 590×g. Following centrifugation, a guide thread in the form of nylon thread (manufactured by Matsuda Ika Kogyo) of total length 5 mm was inserted in the second concavity in such fashion as to penetrate the murine dermal papilla cells and the murine epithelial cells within the culture container. Following insertion thereinto, organ culture was carried out for 16-26 hours within a CO₂ incubator set to have a 5% concentration of CO₂ and set to be at a temperature of 37° C. Following culture, the guide thread was pulled out therefrom in such fashion that the murine dermal papilla cell aggregate and the murine epithelial cell aggregate were not dislodged from the guide thread, and a phase contrast microscope (manufactured by Carl Zeiss) was used to photograph the regenerative hair follicle germ. As a result of determination of whether regenerative hair follicle germ formation had been achieved based on the states of the murine dermal papilla cell aggregate and the murine epithelial cell aggregate, the state of mutual contact between aggregates, and the positional relationship between the guide thread and the respective aggregates, and as a result of calculation of regenerative hair follicle germ plating efficiency (number of regenerative hair follicle germs formed/total number of regenerative hair follicle germs manufactured × 100), because it was found that culture containers for which hole diameter at the second concavity was 0.45 mm displayed higher regenerative hair follicle germ plating efficiency, these were able to produce more stable regenerative hair follicle germs (FIG. 8).

### (6) Evaluation of Regenerative Hair Follicle Germ Organ Inductivity

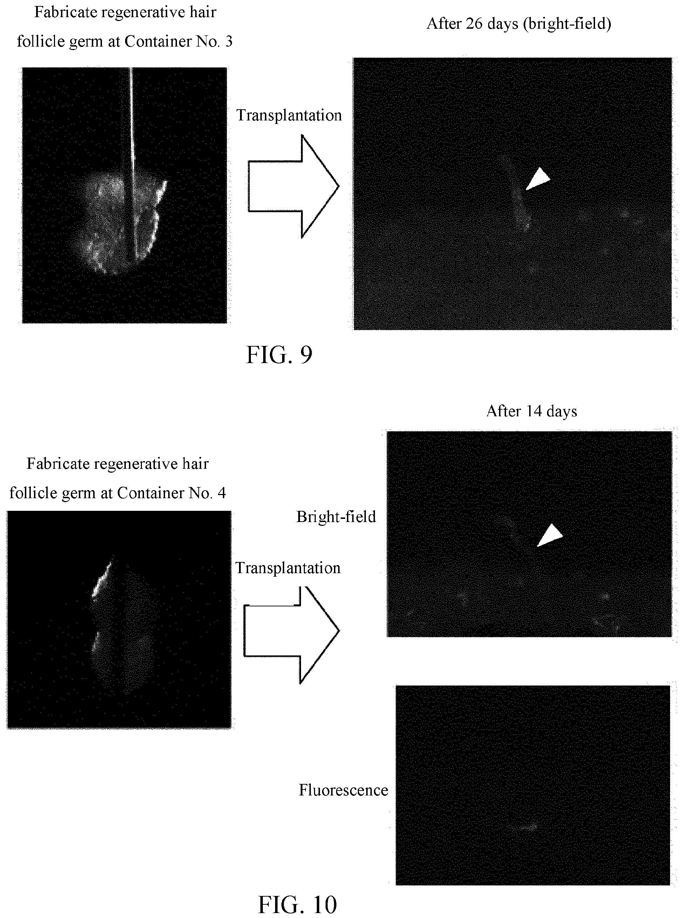

Intracutaneous transplantation of the regenerative hair follicle germ into the skin of a mouse was carried out in accordance with the conventional method. That is, a Balb/c nu/nu mouse (SLC, Inc.) of age 6 to 8 weeks was anesthetized in the usual way, and following disinfection of the dorsum with Isodine, was made to assume a naturally recumbent posture. A V-lance microscalpel (manufactured by Alcon Japan) was used to pierce the skin, the graft which was formed extending from the epidermal layer of the skin to the subdermal layer. The graft extended to a depth of approximately 400 µm in a vertical direction from the body surface, being on the order of approximately 1 mm in the horizontal direction. Sharp Tweezers No. 5 (manufactured by Natsume Seisakusho) were used to cause the regenerative hair follicle germ in which the guide thread was inserted to be inserted therein in such fashion as to cause the epithelial cell component to be directed toward the body surface side of the graft. Transplanted depth was adjusted so as to cause the top portion of the epithelial cell component of the regenerative hair follicle germ to be exposed at the top portion of the graft, the guide thread which was made of nylon thread being located so as to be exposed at the body surface. The guide thread was secured by SteriStrip (manufactured by 3M) to the surface of the skin near the graft, following which Nurseban (manufactured by Sunplanet Co., Ltd.; registered trademark) and surgical tape (manufactured by 3M) were used to protect the graft. 5 to 7 days following transplant, the protective tape (Nurseban and surgical tape) was removed. Survival of the transplanted matter was determined by visual inspection, after which follow-up observation (once every 2 to 4 days) was carried out. Follow-up observation was carried out by using a fluorescence stereomicroscope (manufactured by Carl Zeiss) to inspect and photograph the transplanted location on the anesthetized mouse to evaluate organ inductivity of the regenerative hair follicle. Results are shown in FIG. 9 and FIG. 10. Based on the fact that there was new hair growth at the regenerative hair follicle germs manufactured at Culture Container No. 3 and No. 4, these possessed organ inductivity (white arrowhead).

The present disclosure is not limited to the foregoing embodiments, any number of variations being possible without departing from the gist thereof.

### EXPLANATION OF REFERENCE NUMERALS

- 1: Culture container
- 2: Containing space
- 10: First concavity
- 11: First sidewall portion
- 12: First base portion
- 20: Second concavity
- 21: Second sidewall portion
- 22: Second base portion
- 23: Opening
- 24: Vertex
- 25: Connecting region
- 30: Regenerative hair follicle germ
- 31: Guide thread
- 31a: Tip
- 32: Cells
- 32a: Epithelial cells
- 32b: Mesenchymal cells
- 40: Culture solution
- 50: Cell suspension comprising a plurality of mesenchymal cells
- 60: Cell suspension comprising a plurality of epithelial cells

## Claims

1. A culture container permitting culture of a regenerative hair follicle germ having a guide thread and a plurality of cells which are located at a tip portion of the guide thread, the culture container comprising:
a first concavity; and
a second concavity which opens into a first base portion of the first concavity;
wherein the second concavity has a second base portion which is capable of causing the guide thread to be positioned so as to appear to be located in a central portion of the opening when the opening is viewed from above.

2. The culture container according to claim 1, the culture container being such that
the second base portion is inclined such that diameter thereof decreases as one proceeds toward a lower portion thereof.

3. The culture container according to claim 1 or 2, the culture container being such that
the first base portion is inclined such that diameter thereof decreases as one proceeds toward a lower portion thereof.

4. The culture container according to any of claims 1 through 3, the culture container being such that
the second concavity has a second sidewall portion which is connected to the second base portion; and
the second sidewall portion is inclined such that diameter thereof decreases as one proceeds toward a lower portion thereof.

5. The culture container according to any of claims 1 through 4, the culture container being such that
the second base portion has a conical shape.

6. The culture container according to any of claims 1 through 5, the culture container being such that
the second concavity has a second sidewall portion which is connected to the second base portion; and
length in a vertical direction of the second sidewall portion is greater than diameter of the opening.

7. The culture container according to any of claims 1 through 6, the culture container being such that
the second concavity has a second sidewall portion which is connected to the second base portion, and a connecting region that connects the second base portion and the second sidewall portion; and
depth of the second base portion is less than diameter of the connecting region.

8. The culture container according to any of claims 1 through 7, the culture container being such that
the second concavity has a second sidewall portion which is connected to the second base portion; and
surface roughness of the second base portion is greater than surface roughness of the second sidewall portion.

9. The culture container according to any of claims 1 through 8, the culture container being such that
the second concavity has a second sidewall portion which is connected to the second base portion;
the first base portion, the second sidewall portion, and the second base portion are inclined with respect to the vertical direction; and
angles that first base portion and second base portion make with respect to the vertical direction are greater than an angle that second sidewall portion makes with respect to the vertical direction.

10. The culture container according to any of claims 1 through 9, the culture container being such that
the second concavity has a second sidewall portion which is connected to the second base portion; and
length in the vertical direction of the second sidewall portion is greater than length in the vertical direction of the second base portion.

11. A method for producing the regenerative hair follicle germ, the method for producing the regenerative hair follicle germ comprising:
an operation in which a cell population comprising epithelial cells is made to agglomerate within the second concavity of the culture container according to any of claims 1 through 10;
an operation in which a cell population comprising mesenchymal cells is made to agglomerate within the second concavity;
an operation in which the guide thread in inserted within the second concavity; and
an operation in which culture is carried out while the cell population comprising the agglomerated epithelial cells and the agglomerated mesenchymal cells are made to be in contact without use of a culture support.

12. The production method according to claim 11, the production method being such that
the operation in which the cell population comprising the mesenchymal cells is made to agglomerate is carried out before the operation in which the cell population comprising the epithelial cells is made to agglomerate

13. The production method according to claim 11 or 12, the production method being such that
the operation in which the guide thread is inserted is carried out after the operations in which the cell population comprising the epithelial cells and the cell population comprising the mesenchymal cells are made to agglomerate

14. The production method according to any one of claims 11 through 13, the production method being such that
the cell population comprising the epithelial cells is a cell population comprising bulge region epithelium-derived cells

15. The production method according to any one of claims 11 through 14, the production method being such that
the cell population comprising the mesenchymal cells is a cell population comprising dermal papilla-derived cells.
